(19) Europäisches Patentamt
European Patent Office
Office européen des brevets

(11) Veröffentlichungsnummer: **0 009 554**
**B1**

(12) # EUROPÄISCHE PATENTSCHRIFT

(45) Veröffentlichungstag der Patentschrift: **27.04.83**

(21) Anmeldenummer: **79102566.1**

(22) Anmeldetag: **20.07.79**

(51) Int. Cl.³: **C 07 C 143/822,** A 61 K 31/18, A 61 K 31/44, A 61 K 31/275, C 07 C 143/825, C 07 C 147/08, C 07 C 147/14, C 07 C 149/425, C 07 D 213/64, C 07 D 213/70 // A01N41/06, A01N41/10, A01N43/40, C07C79/35, C07C79/36, C07C93/14, C07C121/48, C07C148/00, C07C149/32, C07C149/42

(54) **Indanyl-Derivate, Verfahren zu ihrer Herstellung und diese Verbindungen enthaltende pharmazeutische Präparate.**

(30) Priorität: **27.07.78 DE 2833202**
**11.06.79 DE 2923937**

(43) Veröffentlichungstag der Anmeldung:
**16.04.80 Patentblatt 80/8**

(45) Bekanntmachung des Hinweises auf die Patenterteilung:
**27.04.83 Patentblatt 83/17**

(84) Benannte Vertragsstaaten:
**AT BE CH DE FR GB IT LU NL SE**

(56) Entgegenhaltungen:
**CH-A-585 705**
**DE-A-2 850 821**
**GB-A-1 472 843**
**US-A-4 086 255**
**US-A-4 087 549**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Schröder, Eberhard, Dr.,**
**Bergengruenstrasse 44 a, D-1000 Berlin 38 (DE)**
Erfinder: **Rufer, Clemens, Dr., Westhofener Weg 14,**
**D-1000 Berlin 38 (DE)**
Erfinder: **Böttcher, Irmgard, Dr., Rodelbahnpfad 5,**
**D-1000 Berlin 28 (DE)**
Erfinder: **Kapp, Joachim-Friedrich, Dr., Ludolfinger**
**Weg 51, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Indanyl-Derivate, Verfahren zu ihrer Herstellung und diese Verbindungen enthaltende pharmazeutische Präparate

Die Erfindung betrifft neue Indanyl-Derivate der allgemeinen Formel I gemäss Anspruch 1, ein Verfahren zu ihrer Herstellung und pharmazeutische Präparate, die diese Substanzen als Wirkstoffe enthalten.

Die Indanyl-Derivate der allgemeinen Formel I gemäss Anspruch 1 haben als Substituenten AR erfindungsgemäss eine Phenylgruppe oder Pyridylgruppe, die gegebenenfalls durch 1 bis 3 Halogenatome (vorzugsweise Fluoratome, Chloratome oder Bromatome), durch 1 bis 3, 1 bis 4 Kohlenstoffatome enthaltende Alkylgruppen (wie zum Beispiel Äthylgruppen, Propylgruppen, Isopropylgruppen oder insbesondere Methylgruppen) oder durch 1 bis 3 Trifluormethylgruppen substituiert sein kann. Geeignete Substituenten AR sind beispielsweise die 2-, 3- oder 4-Fluorphenylgruppe, die 2-, 3- oder 4-Chlorphenylgruppe, die 2-, 3- oder 4-Bromphenylgruppe, die 2,3-, 2,4-, 2,6-, 3,4- oder 3,5-Difluorphenylgruppe, die 2,3-, 2,4-, 2,6-, 3,4- oder 3,5-Dichlorphenylgruppe, die 2-, 3- oder 4-Methylphenylgruppe, die 2-, 3- oder 4-Isopropylphenylgruppe, die 2,3-, 2,4-, 2,6-, 3,4- oder 3,5-Dimethylphenylgruppe, die 2-Chlor-4-fluor-phenylgruppe, die 2-, 3- oder 4-Trifluormethylphenylgruppe, die 2-Chlor-3-methylphenylgruppe, die 3-Chlor-2-methylphenylgruppe, die 2-Chlor-4-methylphenylgruppe, die 4-Chlor-2-methylphenylgruppe, die 2-Chlor-6-methylphenylgruppe, die 2,3-Dichlor-4-methylphenylgruppe, die 4-Chlor-2-fluorphenylgruppe, die 2,4,6-Trichlorphenylgruppe, die 2-Fluor-3-trifluormethylphenylgruppe, die 2-Fluor-4-trifluormethylphenylgruppe, die 3-Fluor-2-trifluormethylphenylgruppe, die 4-Fluor-2-trifluormethylphenylgruppe, die 2-Fluor-6-trifluormethylphenylgruppe, die 2-Pyridylgruppe, die 3-Pyridylgruppe, die 4-Pyridylgruppe, die 5-Chlor-2-pyridylgruppe, die 4-Chlor-3-pyridylgruppe oder die 6-Fluor-3-pyridylgruppe.

Die Indanyl-Derivate der allgemeinen Formel I können als Substituenten Y eine Alkyloximinogruppe (wie zum Beispiel eine Methyloximinogruppe, eine Äthyloximinogruppe, eine Propyloximinogruppe oder eine Butyloximinogruppe) tragen.

Als Substituenten $R_2$ der Indanyl-Derivate kommen beispielsweise der Methylrest, der Äthylrest, der Propylrest, der Isopropylrest, der Butylrest, der Phenylrest, der 3-Nitrophenylrest, der 2-Carboxyphenylrest, der 4-Fluorphenylrest, der 2-Methylphenylrest oder der 2-Chlorphenylrest in Betracht.

Als Substituent $R_1$ der Indanyl-Derivate der allgemeinen Formel I soll beispielsweise der Methylrest, der Äthylrest, der Propylrest, der Isopropylrest, der Butylrest, der Chlormethylrest, der Fluormethylrest oder der Trifluormethylrest verstanden werden.

Erfindungsgemäss können die Gruppe $-\overset{Z}{\overset{|}{C}}H-$ oder $-\overset{Y}{\overset{\|}{C}}-$ der Indanyl-Derivate der allgemeinen Formel I meta- oder paraständig zum Substituenten AR–X des Phenylkerns sein.

Als Acylgruppen, Acyloxygruppen oder Acylaminogruppen der Substituenten V und Z kommen vorzugsweise solche in Betracht, die sich von einer geradkettigen oder verzweigten Alkancarbonsäure (Ameisensäure, Essigsäure, Propionsäure, Buttersäure, Isobuttersäure, Valeriansäure, Capronsäure, Dimethylessigsäure, Trimethylessigsäure usw.) mit 1 bis 6 Kohlenstoffatomen ableiten.

Die Indanyl-Derivate der allgemeinen Formel I gemäss Anspruch 1 sind Herbizide und pharmakologisch wirksame Substanzen, die sich beispielsweise durch eine analgetische, antipyretische, thrombocytenaggregationshemmende, diuretische und insbesondere durch eine antiphlogistische Wirksamkeit auszeichnen. Ein besonderer Vorzug dieser Verbindungen ist darin begründet, dass sie sich durch eine grosse Dissoziation zwischen therapeutischer Wirksamkeit und unerwünschter Nebenwirkung (insbesondere ulcerogener Wirksamkeit) auszeichnen. Darüber hinaus ist bemerkenswert, dass diese Substanzen die Prostaglandinsynthese kaum hemmen.

Die antiphlogistische Wirksamkeit der erfindungsgemässen Substanzen kann mit Hilfe des bekannten Adjuvans-Arthritis-Testes ermittelt werden, der wie folgt durchgeführt wird:

Es werden weibliche und männliche Ratten des Stammes Lewis (LEW) in der Gewichtsspanne zwischen 110 bis 190 g verwendet. Die Tiere erhalten Trinkwasser und Altromin-Pressfutter ad libitum.

Für jede Dosisgruppe werden 10 Ratten eingesetzt.

Mycobacterium butyricum der Firma Difko, Detroit, wird als Reizmittel verwandt. Eine Suspension von 0,5 mg Mycobacterium butyricum in 0,1 ml dünnflüssigem Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myri 53 (85 mg%) in isotonischer Natriumchlorid-Lösung verabreicht.

Versuchsansatz: Die Ratten werden in bezug auf ihr Körpergewicht möglichst gleichmässig in verschiedene Gruppen eingeteilt. Nach plethysmographischer Volumenmessung der rechten Hinterpfote wird in diese subplantar 0,1 ml Adjuvans injiziert. Die rechten Hinterpfoten werden vom 14. Versuchstag bis zum Versuchsende gemessen. Die Versuchsdauer beträgt 3 Wochen.

Bestimmt wird die Abheilung der Hinterpfoten, welche bei der vorgegebenen Dosis erzielt wird.

Eine häufige Komplikation bei der Therapie mit nichtsteroidalen Entzündungshemmern stellt das Auftreten von Magenulcerationen dar. Diese Nebenwirkung kann im Tierversuch nachgewiesen werden, wobei bei vorgegebener Dosis die Anzahl

der beobachteten Läsionen und deren Gesamtfläche ermittelt wird. Der Ulkus-Test wird wie folgt durchgeführt.

Es werden männliche Wistar-Ratten (SPF) verwandt. Die Tiere liegen in einer Gewichtsspanne von 130 ± 10 g. 16 Stunden vor Versuchsbeginn werden die Tiere vom Futter abgesetzt; sie erhalten Wasser ad libitum.

Pro Dosis werden 5 Tiere eingesetzt. Die Substanzen werden einmal oral, in Natriumchlorid gelöst oder als Kristallsuspension unter Zusatz von 85 mg% Myri 53 appliziert.

3 Stunden nach Substanzapplikation injiziert man 1 ml einer 3%igen Lösung des Farbstoffs Diphenylreinblau intravenös und tötet das Tier. Der Magen wird reseziert und mikroskopisch auf Anzahl und Gesamtgrösse von Epithelläsionen und Ulcera, die durch Farbstoffanreicherungen hervortreten, untersucht.

Die nachfolgende Tabelle zeigt die in diesen Testen erhaltenen Ergebnisse der erfindungsgemässen Verbindungen im Vergleich zum vorbekannten Indomethazin (Substanz 1). Aus diesen Ergebnissen ist die Überlegenheit der erfindungsgemässen Verbindungen – insbesondere bezüglich ihrer überlegenen Dissoziation zwischen antiinflammatorischen und ulcerogener Wirksamkeit – ersichtlich.

| Nr. | Substanz | Substanz mg/kg Tier | Adjuvans Arthritis Test % Abheilung rechte Pfote | linke Pfote | Ulcus-Test Substanz in mg/kg Tier | Läsionen Anzahl | Fläche |
|---|---|---|---|---|---|---|---|
| 1 | Indometacin | 4× 4 mg | 50 | 70 | 8 mg | 9,6 | 6,3 |
| 2 | N-(6-Phenoxy-5-indanyl)-methansulfonamid | 4×30 mg | 54 | 74 | 200 mg | 0,4 | 0,2 |
| 3 | N-(6-Phenoxy-5-indanyl)-trifluormethansulfonamid | 4×30 mg | 47 | 81 | 200 mg | 0,4 | 0,2 |
| 4 | N-[6-(4-Fluorphenoxy)-5-indanyl]-methansulfonamid | 4×30 mg | 53 | 76 | 200 mg | 0,2 | 0,1 |
| 5 | N-[6-(4-Chlorphenoxy)-5-indanyl]-methansulfonamid | 4×30 mg | 61 | 86 | 200 mg | 1,4 | 0,7 |
| 6 | N-[6-(4-Chlor-2-methyl-phenoxy)-5-indanyl]-methansulfonamid | 4×30 mg | 48 | 57 | 200 mg | 0,2 | 0,1 |
| 7 | N-[6-(2-Chlorphenoxy)-5-indanyl]-methansulfonamid | 4×30 mg | 51 | 72 | 200 mg | 0,2 | 0,1 |
| 8 | N-(6-Phenylthio-5-indanyl)-methansulfonamid | 4×30 mg | 46 | 75 | 200 mg | 2,0 | 1,0 |
| 9 | N-[6-(2-Pyridylthio)-5-indanyl]-methansulfonamid | 4×30 mg | 54 | 71 | 200 mg | 0,4 | 0,2 |
| 10 | 5-Methylsulfonylamino-6-phenoxy-1-indanon | 4×30 mg | 55 | 69 | 200 mg | 0,2 | 0,1 |
| 11 | N-(5-Phenoxy-6-indenyl)-methansulfonamid | 4×30 mg | 43 | 80 | 200 mg | 0,3 | 0,1 |
| 12 | 5-Methylsulfonylamino-6-phenoxy-2-phenylsulfinyl-1-indanon | 4×30 mg | 50 | 78 | 200 mg | 0,1 | 0,1 |

Die Überlegenheit der erfindungsgemässen Verbindungen, insbesondere ihre überlegene Dissoziation zwischen erwünschter antiinflammatorischer Wirkung und unerwünschter ulcerogener Nebenwirkung, war aufgrund des bekannten Standes der Technik nicht vorhersehbar.

In der Schweizer Patentschrift 585 705 (Riker Lab.) werden Verbindungen beschrieben, die in para-Stellung zur Sulfonamidgruppe eine Nitrogruppe tragen. Diese Nitrogruppe – bekanntlich eine der elektrophilsten Gruppen überhaupt – wird als erfindungswesentlich herausgestellt, da

sie den Verbindungen eine gegenüber den nicht nitrierten Ausgangsprodukten überlegene entzündungshemmende Wirksamkeit verleiht. Vom induktiven Effekt her gesehen und auch von ihrer Raumerfüllung her gesehen, sind die erfindungsgemässen Verbindungen von den Verbindungen des Schweizer Patents so verschieden, dass man völlig andere pharmakologische Wirkungen erwarten sollte.

In der britischen Patentschrift 1 472 843 (Pfitzer) werden Verbindungen beschrieben, die sowohl von ihrer Struktur her als auch von ihrer pharmakologischen Wirksamkeit her (sie haben eine vasodilatorische Wirksamkeit – siehe Seite 1, Zeile 5ff.) völlig verschieden sind.

Im US-Patent 4 086 255 (G.G.I. Moore) und US-Patent 4 087 549 (Tung Ying Shen et al.) werden soweit ausreichend offenbart ebenfalls Verbindungen beschrieben, die von ihrem induktiven Effekt her und ihrer Raumerfüllung her gesehen von den Substanzen unserer Anmeldung völlig verschieden sind. Diese vorbekannten Verbindungen sind zwar antiinflammatorisch wirksam; ein Hinweis darauf, dass diese Verbindungen geringe Nebenwirkungen aufweisen, fehlt aber.

Es ist hinlänglich bekannt, (G. de Stevens Medicinal Chemistry Vol 13 Antiinflammatory Agents Vol 1 1974, Academic Press, New York et al. page 29ff.), dass die antiinflammatorische Wirksamkeit einer Verbindung (wie jede pharmakologische Wirksamkeit) sehr stark von der Struktur der Verbindungen (insbesondere von der Ladungsverteilung in den Molekülen) abhängig ist.

Demzufolge ist sehr unwahrscheinlich, dass die Verbindungen der vorliegenden Anmeldung an die gleichen Rezeptoren gebunden werden wie die vorbekannten Verbindungen.

Die neuen Verbindungen eignen sich in Kombination mit den in der galenischen Pharmazie üblichen Trägermitteln zur Behandlung zum Beispiel von akuter und chronischer Polyarthritis, Neurodermitis, Asthma bronchiale und Heufieber.

Die Herstellung der Arzneimittelspezialitäten erfolgt in üblicher Weise, indem man die Wirkstoffe mit geeigneten Zusätzen, Trägersubstanzen und Geschmackskorriegenzien in die gewünschten Applikationsformen, wie zum Beispiel Tabletten, Dragées, Kapseln, Lösungen, Inhalationsmittel, überführt.

Für die orale Anwendung eignen sich insbesondere Tabletten, Dragées und Kapseln, welche beispielsweise 1 bis 250 mg Wirkstoff und 50 mg bis 2 g pharmakologisch unwirksamen Träger, wie zum Beispiel Laktose, Amylose, Talkum, Gelatine, Magnesiumstearat und ähnliches, sowie die üblichen Zusätze enthalten.

Die neuen Indanyl-Derivate der allgemeinen Formel I können nach an sich bekannten Verfahren hergestellt werden. Ein geeignetes Herstellungsverfahren ist beispielsweise die im Anspruch 7 beschriebene Synthese.

Die Kondensation der Verbindungen der allgemeinen Formel II mit den Sulfonsäurechloriden oder Anhydriden der allgemeinen Formel III gemäss Verfahrensvariante a erfolgt unter den bekannten Bedingungen, beispielsweise indem man die Sulfonsäurechloride in Gegenwart basischer Katalysatoren, wie Natriumkarbonat, Natriumhydroxy, Kaliumbikarbonat, Kaliumkarbonat, Pyridin, Lutidin oder Collidin, mit den Verbindungen der allgemeinen Formel II umsetzt.

Die Oxydation der Methylengruppen zu den entsprechenden Carbonylgruppen gemäss Verfahrensvariante b kann beispielsweise mit Kaliumpermanganat in alkalischer, neutraler oder schwach saurer wässriger Lösung oder mittels einer Lösung von Chrom(VI)-oxid in Eisessig bei einer Temperatur von −10 bis 110 °C durchgeführt werden.

Die Verfahrensvariante c kann beispielsweise in der Weise durchgeführt werden, dass man die Ausgangsverbindungen in einem aprotischen Lösungsmittel (zum Beispiel Pyridin, Dimethylformamid, Hexamethylphosphorsäuretriamid, Dioxan, Tetrahydrofuran) mit einem Alkalimetallhydrid oder Alkalimetallamid (wie zum Beispiel Natriumhydrid oder Lithiumdiisopropylamid) umsetzt und auf das erhaltene Reaktionsgemisch das gewünschte Disulfid einwirken lässt. Diese Reaktion wird vorzugsweise bei einer Reaktionstemperatur von −60 bis +20 °C durchgeführt.

Die sich gegebenenfalls anschliessende Oxydation der Thioverbindungen zu den Sulfoxyden oder Sulfonen der allgemeinen Formel I erfolgt nach an sich bekannten Arbeitsmethoden.

Bei dieser Reaktion kann man als Oxydationsmittel beispielsweise Persäuren, wie Peressigsäure, Perbenzoesäure oder m-Chlorperbenzoesäure, Wasserstoffperoxyd, Chinone, wie 2,3-Dichlor-5,6-dicynobenzochinon, IV- bis VIIwertige Metalloxyde oder -salze, wie Blei(IV)-oxyd, Mangan(IV)-oxyd, Chrom(VI)-oxyd, Cer(IV)-sulfat, Kaliumchromat, Kaliumdichromat, Kaliumpermanganat oder oxydierende Halogenverbindungen, wie Jod, Natriumperjodat, N-Bromsucciniimid, N-Chlorsucciniimid oder Natriumchlorid verwendet.

Benutzt man für diese Oxydation Wasserstoffperoxyd oder Metalloxyde oder -salze, so ist es zweckmässig, die Oxydation in Gegenwart von Säuren durchzuführen. Geeignete Säuren sind Mineralsäuren, wie Chlorwasserstoff oder Schwefelsäure, oder niedere Carbonsäuren, wie Essigsäure oder Propionsäure.

Als Lösungsmittel kann man für diese Reaktion sowohl protische als auch aprotische inerte Lösungsmittel verwenden. Geeignete Lösungsmittel sind beispielsweise niedere Carbonsäuren, wie Essigsäure oder Propionsäure, tert.-Alkohole, wie tert.-Butanol, Ketone, wie Aceton, Methyläthylketon oder Cyclohexanon, Äther, wie Diäthyläther, Diisopropyläther, Tetrahydrofuran, Dioxan oder Glykoldimethyläther, Kohlenwasserstoffe, wie Benzol oder Toluol oder chlorierte Kohlenwasserstoffe, wie Methylenchlorid, Chloroform, Tetrachlorkohlenstoff, Tetrachloräthan oder Chlorbenzol. Zur Herstellung von Sulfonen der allgemeinen Formel I verwendet man als Lösungsmittel vorzugsweise Essigsäure. Die Herstellung von Sulfoxyden erfolgt vorzugsweise in Aceton als Lösungsmittel.

Die Verfahrensvariante d wird vorzugsweise in einem polaren Lösungsmittel (beispielsweise einem niederen Alkohol, wie Methanol, Äthanol oder Isopropanol, oder einer niederen Carbonsäure, wie Essigsäure) bei einer Reaktionstemperatur von −10 bis 110 °C durchgeführt.

Die Verfahrensvariante e kann in der Weise durchgeführt werden, dass man die Ausgangsverbindungen mit komplexen Metallhydriden, wie zum Beispiel Natriumborhydrid (in einem niederen Alkohol, wie Methanol, Äthanol oder Isopropanol als Lösungsmittel) gegebenenfalls unter Zusatz von Bortrifluorid oder Trifluoressigsäure oder Lithiumaluminiumhydrid (in einem polaren Äther wie Tetrahydrofuran oder Dioxan als Lösungsmittel) reduziert.

Anderseits kann man diese Variante aber auch in der Weise durchführen, dass man die Ausgangsverbindungen in Gegenwart von Hydrierungskatalysatoren (zum Beispiel Raney-Nikkel, Platinoxyd, Palladium-Tierkohle) mit Wasserstoff bei 1 bis 150 atm. Druck hydriert.

Die Verfahrensvariante f wird unter den Bedingungen durchgeführt, welche man üblicherweise zur Dehydratisierung verwendet. Geeignete Methoden sind beispielsweise die Dehydratisierung, die Umsetzung der Verbindungen mit Säuren (zum Beispiel Toluolsulfonsäure, Schwefelsäure, Polyphosphorsäure) oder wasserentziehenden Mitteln (zum Beispiel Kieselgel, Phosphorpentoxyd) in inerten Lösungsmitteln (niedere Ketone wie Aceton, Äther wie Tetrahydrofuran oder Dioxan oder aromatischen Kohlenwasserstoffen wie Benzol, Toluol oder Xylol).

Die Ausgangsverbindungen für das erfindungsgemässe Verfahren gemäss Anspruch 8 sind bekannt oder können in an sich bekannter Weise hergestellt werden.

So kann man beispielsweise die Verbindungen der allgemeinen Formel II durch Kondensation von Verbindungen der allgemeinen Formel IV mit Verbindungen der allgemeinen Formel III und anschliessender Reduktion der erhaltenen Nitroverbindungen der allgemeinen Formel V herstellen.

ArX H +

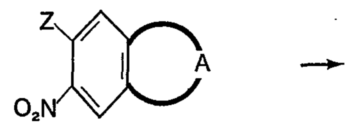

(III) (IV)

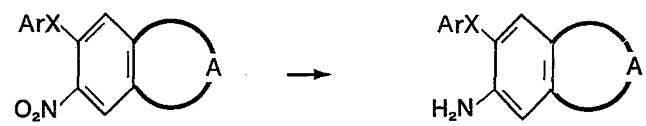

(V) (II)

ArX und A haben die im Anspruch 1 genannte Bedeutung, und Z bedeutet ein Chlor-, Brom- oder Jodatom.

Die Bedingungen, unter denen diese Ausgangsverbindungen synthetisiert werden können, sind am Beispiel ausgewählter Vertreter in den nachfolgenden Ausführungsbeispielen beschrieben. Die nachfolgenden Ausführungsbeispiele dienen zur Erläuterung des erfindungsgemässen Verfahrens.

Beispiel 1

a) 12,1 g 5-Brom-6-nitroindan, 0,75 g Kupfer(I)-chlorid, 17,3 g Kaliumcarbonat und 18,4 g Phenol in 200 ml absolutem Pyridin werden 3 Stunden am Rückfluss gekocht. Man engt im Vakuum ein, versetzt den Rückstand mit Chloroform und saugt vom Unlöslichen ab. Die Mutterlauge wird je 3mal mit 1n Natronlauge und 1n Salzsäure ausgeschüttelt und im Vakuum eingeengt. Nach Destillation im Vakuum werden 11,5 g 5-Nitro-6-phenoxyindan vom Siedepunkt $Kp_0$,03 163 bis 165 °C erhalten. Schmelzpunkt: 41 °C (Hexan).

b) Eine Lösung von 17,7 g 5-Nitro-6-phenoxyindan in 500 ml Methanol wird in Gegenwart von 15 g Raney-Nickel in 4 Stunden bei 70 at hydriert. Man filtriert vom Katalysator ab, engt im Vakuum ein und kristallisiert aus wässrigem Äthanol um. Es werden 12,9 g 6-Phenoxy-5-indanylamin vom Schmelzpunkt 62 °C erhalten.

c) In eine Lösung von 7,8 g 6-Phenoxy-5-indanylamin in 50 ml absolutem Pyridin werden bei 0 °C in 10 Minuten 4 ml Methansulfonchlorid eingetropft. Man rührt 3 Stunden bei 0 °C und 16 Stunden bei Raumtemperatur und engt im Vakuum ein. Der Rückstand wird in Chloroform aufgenommen, 3mal mit 1n Salzsäure ausgeschüttelt und im Vakuum eingeengt. Dann kristallisiert man aus wässrigem Äthanol um und erhält 9,5 g N-(6-Phenoxy-5-indanyl)-methansulfonamid vom Schmelzpunkt 130 °C.

Beispiel 2

Analog Beispiel 1 werden ausgehend von 5-Brom-6-nitroindan und 4-Chlorphenol erhalten:

a) 5-(4-Chlorphenoxy)-6-nitroindan vom Siede-

punkt $Kp_{0,03}$ 165 bis 68 °C und vom Schmelzpunkt 67 °C (Hexan),

b) 6-(4-Chlorphenoxy)-5-indanylamin vom Schmelzpunkt 66 °C,

c) N-[6-(4-Chlorphenoxy)-5-indanyl]-methansulfonamid vom Schmelzpunkt 57 °C.

Beispiel 3

Analog Beispiel 1 werden ausgehend von 5-Brom-6-nitroindan und p-Kresol erhalten:

a) 5-Nitro-6-(4-tolyloxy)-indan vom Siedepunkt $Kp_{0,03}$ 168 bis 73 °C und vom Schmelzpunkt 58 °C (Hexan).

b) 6-(4-Tolyloxy)-5-indanylamin als Öl.

c) N-[6-(4-Tolyloxy)-5-indanyl]-methansulfonamid vom Schmelzpunkt 139 °C.

Beispiel 4

Analog Beispiel 1 werden ausgehend von 5-Brom-6-nitroindan und 4-Fluorphenol erhalten:

a) 5-(4-Fluorphenoxy)-6-nitroindan vom Siedepunkt und vom Schmelzpunkt 63 °C (Hexan),

b) 6-(4-Fluorphenoxy)-5-indanylamin vom Schmelzpunkt 72 °C,

c) N-[6-(4-Fluorphenoxy)-5-indanyl]-methansulfonamid vom Schmelzpunkt 100 °C.

Beispiel 5

Analog Beispiel 1 werden ausgehend von 5-Brom-6-nitroindan und 3-Trifluormethylphenol erhalten:

a) 6-Nitro-5-(3-trifluormethyl-phenoxy)-indan vom Siedepunkt $Kp_{0,03}$ 155 bis 63 °C und vom Schmelzpunkt 69 °C (Hexan),

b) 6-(3-Trifluormethyl-phenoxy)-5-indanylamin als Öl,

c) N-[6-(3-Trifluormethylphenoxy)-5-indanyl]-methansulfonamid vom Schmelzpunkt 81 °C.

Beispiel 6

Analog Beispiel 1 werden ausgehend von 5-Brom-6-nitro-indan und 4-Chlor-2-methylphenol erhalten:

a) 5-(4-Chlor-2-methylphenoxy)-6-nitroindan vom Siedepunkt $Kp_{0,03}$ 187 bis 90 °C und vom Schmelzpunkt 61 °C (Hexan),

b) 6-(4-Chlor-2-methylphenoxy)-5-indanylamin als Öl,

c) N-[6-(4-Chlor-2-methyl-phenoxy)-5-indanyl]-methansulfonamid vom Schmelzpunkt 116 °C.

Beispiel 7

Analog Beispiel 1 werden ausgehend von 5-Brom-6-nitroindan und 2-Chlorphenol erhalten:

a) 5-(2-Chlorphenoxy)-6-nitroindan vom Siedepunkt $Kp_{0,03}$ 175 bis 78 °C,

b) 6-(2-Chlorphenoxy)-5-indanylamin als Öl,

c) N-[6-(2-Chlorphenoxy)-5-indanyl]-methansulfonamid vom Schmelzpunkt 120 °C.

Beispiel 8

Analog Beispiel 1 werden ausgehend von 5-Brom-6-nitroindan und 3-Chlorphenol erhalten:

a) 5-(3-Chlorphenoxy)-6-nitroindan vom Siedepunkt $Kp_{0,03}$ 176 bis 82 °C,

b) 6-(3-Chlorphenoxy)-5-indanylamin als Öl,

c) N-[6-(3-Chlorphenoxy)-5-indanyl]-methansulfonamid vom Schmelzpunkt 109 bis 111 °C.

Beispiel 9

Analog Beispiel 1 werden ausgehend von 5-Brom-6-nitroindan und 2-Fluorphenol erhalten:

a) 5-(2-Fluorphenoxy)-6-nitroindan vom Siedepunkt $Kp_{0,03}$ 155 bis 65 °C und vom Schmelzpunkt 47 °C,

b) 6-(2-Fluorphenoxy)-5-indanylamin als Öl,

c) N-[6-(2-Fluorphenoxy)-5-indanyl]-methansulfonamid vom Schmelzpunkt 78 °C.

Beispiel 10

Analog Beispiel 1 werden ausgehend von 5-Brom-6-nitroindan und 2-Chlor-4-fluorphenol erhalten:

a) 5-(2-Chlor-4-fluorphenoxy)-6-nitroindan als Öl,

b) 6-(2-Chlor-4-fluorphenoxy)-5-indanylamin vom Schmelzpunkt 63 °C,

c) N-[6-(2-Chlor-4-fluorphenoxy)-5-indanyl]-methansulfonamid vom Schmelzpunkt 90 °C.

Beispiel 11

Analog Beispiel 1 werden ausgehend von 5-Brom-6-nitroindan und 3,4-Dichlorphenol erhalten:

a) 5-(3,4-Dichlorphenoxy)-6-nitroindan nach Kieselgel-Säulenreinigung (System: Tetrachlormethan/Essigsäureäthylester 30/1) als Öl,

b) 6-(3,4-Dichlorphenoxy)-5-indanylamin vom Schmelzpunkt 84 °C,

c) N-[6-(3,4-Dichlorphenoxy)-5-indanyl]-methansulfonamid vom Schmelzpunkt 135 °C.

Beispiel 12

Analog Beispiel 1 werden ausgehend von 5-Brom-6-nitroindan und 4-Bromphenol erhalten:

a) 5-(4-Bromphenoxy)-6-nitroindan vom Siedepunkt $Kp_{0,03}$ 183 bis 85 °C,

b) 6-(4-Bromphenoxy)-5-indanylamin vom Schmelzpunkt 66 °C,

c) N-[6-(4-Bromphenoxy)-5-indanyl]-methansulfonamid vom Schmelzpunkt 118 °C.

Beispiel 13

Analog Beispiel 1 werden ausgehend von 5-Brom-6-nitroindan und 2,4-Dichlorphenol erhalten:

a) 5-(2,4-Dichlorphenoxy)-6-nitroindan, Reinigung über eine Kieselgel-Säule (System: Tetrachlormethan/Essigäthylester, 30/1) als Öl,

b) 6-(2,4-Dichlorphenoxy)-5-indanylamin als Öl,

c) N-[6-(2,4-Dichlorphenoxy)-5-indanyl]-methansulfonamid vom Schmelzpunkt 90 °C.

Beispiel 14

Analog Beispiel 1 werden ausgehend von 5-Brom-6-nitroindan und o-Kresol erhalten:

a) 5-Nitro-6-(2-Tolyloxy)indan vom Siedepunkt $Kp_{0,05}$ 163 bis 166 °C,

b) 6-(2-Tolyloxy)-5-indanylamin als Öl,

c) N-[6-(2-Tolyloxy)-5-indanyl]methansulfonamid vom Schmelzpunkt 92 °C.

Beispiel 15

Analog Beispiel 1 werden ausgehend von 5-Brom-6-nitroindan und 3-Fluorphenol erhalten

a) 5-(3-Fluorphenoxy)-6-nitroindan vom Siedepunkt $Kp_{0,03}$ 155 bis 63 °C,

b) 6-(3-Fluorphenoxy)-5-indanylamin vom Schmelzpunkt 49 °C,

c) N-[6-(3-Fluorphenoxy)-5-indanyl]-methansulfonamid vom Schmelzpunkt 102 °C.

Beispiel 16

a) 14,6 g 5-Brom-6-nitroindan, 1,2 g Kupfer(I)-chlorid, 12,4 ml Thiophenol und 8,4 g Kaliumcarbonat in 150 ml absolutem Pyridin werden 3 Stunden unter Stickstoff am Rückfluss gekocht. Man engt im Vakuum ein und arbeitet, wie in Beispiel 1a beschrieben, den Ansatz auf. Es wird aus Essigsäureäthylester umkristallisiert, und man erhält 8,1 g 5-Phenylthio-6-nitroindan vom Schmelzpunkt 112 °C.

b) In eine Lösung von 9 g 5-Phenylthio-6-nitroindan in 160 ml Äthanol und 6,6 ml Hydrazinhydrat werden in der Siedehitze 5 g Raney-Nickel eingetragen und 1¼ Stunde am Rückfluss gekocht. Man filtriert vom Katalysator ab, engt bis zur Kristallisation ein und erhält 7,1 g 6-Phenylthio-5-indanylamin vom Schmelzpunkt 80 °C.

c) Das erhaltene Produkt wird, wie im Beispiel 1c beschrieben, zum N-(6-Phenylthio-5-indanyl)-methansulfonamid vom Schmelzpunkt 115,5 °C umgesetzt.

Beispiel 17

Analog Beispiel 16 werden ausgehend von 5-Brom-6-nitroindan und 4-tert.-Butylbenzolthiol erhalten:

a) 5-(4-tert.-Butylphenylthio)-6-nitroindan vom Schmelzpunkt 94 °C,

b) 6-(4-tert.-Butylphenylthio)-5-indanylamin vom Schmelzpunkt 92 °C,

c) N-[6-(4-tert.-Butylphenylthio)-5-indanyl]-methansulfonamid vom Schmelzpunkt 116 °C.

Beispiel 18

Analog Beispiel 16 werden ausgehend von 5-Brom-6-nitroindan und 4-Fluor-thiophenol erhalten:

a) 5-(4-Fluorphenylthio)-6-nitroindan vom Schmelzpunkt 106 °C,

b) 6-(4-Fluorphenylthio)-5-indanylamin vom Schmelzpunkt 60 °C,

c) N-[6-(4-Fluorphenylthio)-5-indanyl]-methansulfonamid vom Schmelzpunkt 139 °C.

Beispiel 19

a) 2,4 g 5-Brom-6-nitroindan, 2,25 g Kalium-tert.-butylat und 2,9 g 4-Chlor-thiophenol werden in 30 ml absolutem Dimethylformamid unter Stickstoff 3 Stunden auf 60 °C erwärmt. Es wird im Vakuum eingeengt, mit Essigsäureäthylester versetzt und 3mal mit 2n Natronlauge ausgeschüttelt. Man engt erneut ein und reinigt den Rückstand auf einer Kieselgel-Säule (System: Tetrachlormethan/Essigsäureäthylester 25/1) und kristallisiert aus Ethanol um. Es wurden 1,2 g 5-(4-Chlorphenylthio)-6-nitroindan vom Schmelzpunkt 118 °C erhalten.

b) Analog Beispiel 16b wird daraus erhalten: 6-(4-Chlorphenylthio)-5-indanylamin vom Schmelzpunkt 63 °C,

c) Analog Beispiel 1c wird daraus erhalten: N-[6-(4-Chlorphenylthio)-5-indanyl]-methansulfonamid vom Schmelzpunkt 109 °C.

Beispiel 20

a) 4,8 g 5-Brom-6-nitroindan, 4,5 g Kalium-tert.-butylat, 4,4 g 2-Pyridinthiol in 50 ml absolutem Dimethylformamid werden 3 Stunden unter Stickstoff auf 80 °C erwärmt. Man engt im Vakuum ein, versetzt den Rückstand mit Essigsäureäthylester und schüttelt 4mal mit Wasser aus. Die Essigsäureäthylester-Phase wird eingeengt und der Rückstand auf einer Kieselgel-Säule gereinigt (System: Cyclohexan/Essigsäureäthylester, 4/1). Nach Kristallisation aus Ethanol werden 3,3 g 5-Nitro-6-(2-pyridylthio)indan vom Schmelzpunkt 74 °C erhalten.

b) Analog Beispiel 16b wird daraus erhalten: 6-(2-Pyridylthio)-5-indanylamin vom Schmelzpunkt 126 °C.

c) Analog Beispiel 1c wird daraus erhalten: N-[6-(2-Pyridylthio)-5-indanyl]-methansulfonamid vom Schmelzpunkt 141 °C.

Beispiel 21

a) 7,2 g 5-Brom-6-nitroindan und 3,9 g 4-Pyridinthiol werden in 120 ml Dimethylsulfoxyd mit 3,6 g Natriumhydrogencarbonat 7 Stunden unter Stickstoff auf 50 °C erwärmt. Es wird eingeengt, der Rückstand in Chloroform/Wasser gelöst und 3mal mit Wasser ausgeschüttelt. Man engt ein und reinigt über eine Kieselgel-Säule (System: Cyclohexan/Essigester 1/1). Nach Kristallisation aus Ethanol werden 2 g 5-(4-Pyridylthio)-6-nitroindan vom Schmelzpunkt 113 °C erhalten.

b) Analog Beispiel 16b wird daraus erhalten: 6-(4-Pyridylthio)-5-indanylamin vom Schmelzpunkt 140 °C.

c) Analog Beispiel 1c wird daraus erhalten: N-[6-(4-Pyridylthio)-5-indanyl]-methansulfonamid vom Schmelzpunkt 156 °C.

Beispiel 22

Analog Beispiel 1 werden ausgehend von 5-Brom-6-nitroindan und 3-Chlorpyridin erhalten:

a) 6-(3-Pyridyloxy)-5-nitroindan als Öl,

b) 6-(3-Pyridyloxy)-5-indanylamin vom Schmelzpunkt 118 °C,

c) N-[6-(3-Pyridyloxy)-5-indanyl]-methylsulfonamid vom Schmelzpunkt 126 °C.

Beispiel 23

1,1 g 6-Phenoxy-5-indanylamin werden in 15 ml absolutem Pyridin gelöst und bei 0 °C in 10 Minuten mit 1,6 ml Trifluormethansulfonsäureanhydrid in 5 ml absolutem Benzol versetzt. Man rührt 3 Stunden bei 0 °C und 16 Stunden bei Raumtemperatur, engt im Vakuum ein, nimmt den Rückstand in Chloroform auf und schüttelt 3mal mit 1n Salzsäure aus. Dann engt man die Chloroform-Phase im Vakuum ein, reinigt den Rückstand auf einer Kieselgel-Säule (System: Chloroform) und kristallisiert aus Hexan um. Es werden 0,74 g N-(6-Phenoxy-5-indanyl)-trifluormethansulfonamid vom Schmelzpunkt 90 °C erhalten.

Beispiel 24

Analog Beispiel 23 wird aus 6-Phenylthio-5-indanylamin erhalten:
N-(6-Phenylthio-5-indanyl)-trifluormethansulfonamid, Schmelzpunkt 67 °C.

Beispiel 25

Analog Beispiel 23 wird aus 6-(4-Fluorphenoxy)-5-indanylamin erhalten:
N-[6-(4-Fluorphenoxy)-5-indanyl]-trifluormethansulfonamid, Schmelzpunkt 123 °C.

Beispiel 26

Analog Beispiel 23 wird aus 4-Chlorphenoxy-5-indanylamin erhalten:
N-[6-(4-Chlorphenoxy)-5-indanyl]-trifluormethansulfonamid, Schmelzpunkt 139 °C.

Beispiel 27

Analog Beispiel 23 wird aus 6-Phenoxy-5-indanylamin mit Chlormethansulfonsäureanhydrid erhalten:
N-(6-Phenoxy-5-indanyl)-chlormethansulfonamid vom Schmelzpunkt 73 °C.

Beispiel 28

Analog Beispiel 23 wird aus 6-Phenoxy-5-indanylamin mit Äthansulfonsäureanhydrid erhalten:
N-(6-Phenoxy-5-indanyl)-äthansulfonamid vom Schmelzpunkt 89 °C.

Beispiel 29

a) 11 g 5-Nitro-6-phenoxyindan und 17,3 g Bis-dimethylamino-tert.-butoxymethan werden in 60 Minuten auf 155 °C erwärmt und 60 Minuten bei dieser Temperatur gehalten, wobei tert.-Butanol abdestilliert wird. Danach wird im Vakuum eingeengt, mit 50 ml Ethanol versetzt und abgesaugt. 9,2 g 1-Dimethylaminomethylen-5-nitro-6-phenoxyindan vom Schmelzpunkt 97 °C werden erhalten.

b) 6,2 g dieses Enamins werden in 100 ml Chloroform gelöst und bei −35 °C ozonisiert. Nach Filtration über 30 g Kieselgel mit Chloroform, Einengen und Kristallisation aus 30 ml Ethanol werden 3,8 g 5-Nitro-6-phenoxy-1-indanon vom Schmelzpunkt 105 °C erhalten.

c) 1,58 g dieses Nitroketons werden in 20 ml Ethanol und 10 ml Dioxan gelöst. Die Lösung wird mit 0,74 g Hydrazinhydrat versetzt, und etwa 1,5 g Raney-Nickel (in Ethanol aufgeschlemmt) werden bei 35 °C portionsweise eingetragen. Nach 60 Minuten am Rückfluss wird gekühlt, filtriert und eingeengt. Umkristallisation aus Ethanol ergibt 1,22 g 5-Amino-6-phenoxy-1-indanon vom Schmelzpunkt 170 °C.

d) 1,2 g dieses Aminoketons werden in 12 ml Pyridin bei 0 °C im Vakuum eingeengt, der Rückstand mit Eiswasser versetzt und abgesaugt. Der Niederschlag wird in verdünnter Natronlauge gelöst und die filtrierte Lösung mit Salzsäure angesäuert. Absaugen und Umkristallisieren aus Ethanol ergibt 1,35 g 5-Methylsulfonylamino-6-phenoxy-1-indanon vom Schmelzpunkt 175 °C.

Beispiel 30

Analog Beispiel 29 werden ausgehend von 6-(4-Chlorphenoxy)-5-nitroindan erhalten:

a) 6-(4-Chlorphenoxy)-1-dimethylaminomethylen-5-nitroindan vom Schmelzpunkt 117 °C,

b) 6-(4-Chlorphenoxy)-5-nitro-1-indanon vom Schmelzpunkt 131 °C,

c) 5-Amino-6-(4-chlorphenoxy)-1-indanon vom Schmelzpunkt 169 °C,

d) 6-(4-Chlorphenoxy)-5-methylsulfonylamino-1-indanon vom Schmelzpunkt 185 °C.

Beispiel 31

Analog Beispiel 29 werden ausgehend von 6-(4-Fluorphenoxy)-5-nitroindan erhalten:

a) 1-Dimethylaminomethylen-6-(4-fluorphenoxy)-5-nitroindan vom Schmelzpunkt 128 °C,

b) 6-(4-Fluorphenoxy)-5-nitro-1-indanon vom Schmelzpunkt 150 °C,

c) 5-Amino-6-(4-fluorphenoxy)-1-indanon vom Schmelzpunkt 167 °C,

d) 6-(4-Fluorphenoxy)-5-methylsulfonylamino-1-indanon vom Schmelzpunkt 144 °C.

Beispiel 32

Analog Beispiel 29 werden ausgehend von 6-(3-Chlorphenoxy)-5-nitroindan erhalten:

a) 6-(3-Chlorphenoxy)-1-dimethylaminomethylen-5-nitroindan vom Schmelzpunkt 78 °C,

b) 6-(3-Chlorphenoxy)-5-nitro-1-indanon vom Schmelzpunkt 92 °C,

c) 5-Amino-6-(3-chlorphenoxy)-1-indanon vom Schmelzpunkt 162 °C,

d) 6-(3-Chlorphenoxy)-5-methylsulfonylamino-1-indanon vom Schmelzpunkt 129 °C.

Beispiel 33

Analog Beispiel 29 werden ausgehend von 6-(2-Fluorphenoxy)-5-nitroindan erhalten:

a) 1-Dimethylaminomethylen-6-(2-fluorphenoxy)-5-nitroindan vom Schmelzpunkt 122 °C,

b) 6-(2-Fluorphenoxy)-5-nitro-1-indanon vom Schmelzpunkt 104 °C,

c) 5-Amino-6-(2-fluorphenoxy)-1-indanon vom Schmelzpunkt 164 °C,

d) 6-(2-Fluorphenoxy)-5-methylsulfonylamino-1-indanon vom Schmelzpunkt 119 °C.

Beispiel 34

3,17 g 5-Methylsulfonylamino-6-phenoxy-1-indanon werden in Pyridin bei −40 °C mit 27 ml einer 10%igen Lösung von Lithium-diisopropylamid in Hexan versetzt. Nach 20 Minuten bei −35 °C werden 4,4 g Diphenyldisulfid in 10 ml Pyridin hinzugetropft. Nach 1 Stunde bei −10 °C und 2 Stunden bei 20 °C werden 10 ml 2-Propanol hinzugetropft. Nach Einengen im Vakuum wird in Wasser aufgenommen, filtriert, angesäuert und

mit Chloroform extrahiert. Einengen und Chromatografie des Rückstandes über 240 g Kieselgel mit Chloroform als Elutionsmittel ergibt zunächst 400 mg 5-Methylsulfonylamino-6-phenoxy-2,2-bis-(phenylthio)-1-indanon vom Schmelzpunkt 162 °C und danach 2 g 5-Methylsulfonyl-amino-6-phenoxy-2-phenylthio-1-indanon vom Schmelzpunkt 86 °C.

Beispiel 35

1,7 g 5-Methylsulfonylamino-6-phenoxy-2-phenylthio-1-indanon werden in 20 ml Methanol gelöst und bei 20 °C mit 4 ml einer 1n Lösung von Perselensäure (Lit.: J. Drabowicz, M. Mikolajczyk, Synthesis 1978, 758) in Methanol versetzt. Nach 30 Minuten werden 30 ml Wasser hinzugegeben, Methanol im Vakuum entfernt und das Kristallisat abgesaugt. Es werden 1,7 g 5-Methylsulfonyl-amino-6-phenoxy-2-phenylsulfinyl-1-indanon vom Schmelzpunkt 120 °C erhalten.

Beispiel 36

600 mg 5-Methylsulfonylamino-6-phenoxy-2-phenylthio-1-indanon werden in 5 ml Essigsäure mit 2 ml 30%igem Wasserstoffperoxid 30 Minuten bei 90 °C gehalten. Danach wird auf 20 °C gekühlt, 15 ml Eiswasser werden hinzugefügt und das Kristallisat abgesaugt. Umkristallisation aus Ethanol ergibt 400 mg 5-Methylsulfonylamino-6-phenoxy-2-phenylsulfonyl-1-indanon vom Schmelzpunkt 180 °C.

Beispiel 37

4,12 g 5-Methylsulfonylamino-6-phenoxy-1-indanon werden in 45 ml Methanol und 13 ml 1n Natronlauge gelöst. Bei 5 °C werden 0,98 g Natriumborhydrid hinzugegeben. Nach 16 Stunden bei 20 °C wird eingeengt, mit Eiswasser versetzt, mit Salzsäure neutralisiert und mit Chloroform extrahiert. Waschen der Chloroformlösung mit Wasser, Einengen und Umkristallisation des Rückstandes aus Toluol ergibt 3,3 g 5-Methylsulfonylamino-6-phenoxy-1-indanol vom Schmelzpunkt 96 °C.

Beispiel 38

1,97 g 5-Methylsulfonylamino-6-phenoxy-1-indanon werden in 40 ml Methanol und 13 ml Wasser mit 1,1 g Natriumacetattrihydrat und 1,05 g Hydroxylamin-hydrochlorid 6 Stundcn gekocht. Abkühlen und Absaugen ergibt 1,8 g N-(1-Hydroximino-6-phenoxy-5-indanyl)-methansulfonamid vom Schmelzpunkt 216 °C.

Beispiel 39

3,17 g 5-Methylsulfonylamino-6-phenoxy-1-indanon werden wie in Beispiel 8 beschrieben mit Methoxyamin-hydrochlorid behandelt. Es werden 2,8 g N-(1-Methoximino-6-phenoxy-5-indanyl)-methansulfonamid vom Schmelzpunkt 178 °C erhalten.

Beispiel 40

3,17 g 5-Methylsulfonylamino-6-phenoxy-1-indanon werden in 80 ml Methylglykol bei 80 °C mit 2 g p-Toluolsulfonsäurehydrazid mit wenigen Tropfen Salzsäure 30 Minuten gerührt. Kühlen und Absaugen ergibt 3,74 g N-[1-(4-Toluolsulfonylhydrazono)-6-phenoxy-5-indanyl]-methansulfonamid vom Schmelzpunkt 252 °C.

Beispiel 41

3,33 g N-(1-Hydroximino-6-phenoxy-5-indanyl)-methansulfonamid werden in 100 ml Methanol in Gegenwart von Ammoniak und 0,5 g Nickel bei 90 °C und 75 at hydriert. Nach Filtrieren und Einengen wird der Rückstand über eine Kieselgelsäule gereinigt (System: Chloroform/Methanol 1/1). Aufnehmen in Natronlauge und Ansäuern auf pH 6 mit Essigsäure ergibt 715 mg 5-Methylsulfonylamino-6-phenoxy-1-indanylamin als Acetat vom Schmelzpunkt 175 °C.

Beispiel 42

3,58 g 5-Methylsulfonylamino-6-phenoxy-2-phenylthio-1-indanon werden in 35 ml Methanol und 9 ml 1n Natronlauge gelöst. Bei 5 °C werden 680 mg Natriumborhydrid portionsweise hinzugefügt. Nach 16 Stunden bei 20 °C wird mit 22 ml 1n Salzsäure auf pH 8,2 gestellt und das ausgefallene Kristallisat (3,50 g) abgesaugt. Chromatographie über 120 g Kieselgel mit Chloroform ergibt zunächst 2,5 g cis-5-Methyl-sulfonylamino-6-phenoxy-2-phenylthio-1-indanol vom Schmelzpunkt 135 °C.

Beispiel 43

3,2 g 5-Methylsulfonylamino-6-phenoxy-1-indanol werden 4 Stunden in 32 ml Aceton mit 0,6 g p-Toluolsulfonsäure gerührt. Nach Einengen im Vakuum wird in Chloroform aufgenommen und über 50 g Kieselgel chromatografiert. Zunächst werden 1,47 g N-(5-Phenoxy-5-indenyl)-methansulfonamid vom Schmelzpunkt 154 °C, danach 0,27 g Methansulfonsäure-N-(5-methylsulfonylamino-6-phenoxy-1-indenyl)-amid vom Schmelzpunkt 194 °C erhalten.

Beispiel 44

a) 47,3 g 5-Fluor-1-indanon werden bei 0 bis −5 °C mit 220 ml rauchender Salpetersäure behandelt (3 Stunden). Es wird auf Eiswasser gegeben, mit Chloroform extrahiert, und die Chloroformphase wird neutralgewaschen und eingeengt. Umkristallisation des Rückstandes aus Ethanol ergibt 20,4 g 5-Fluor-6-nitro-1-indanon vom Schmelzpunkt 89 °C.

b) 20,3 g dieser Verbindung werden in 130 ml Dimethylsulfoxid 3 Stunden bei 50 °C mit 9,8 g Phenol und Natriumhydrogencarbonat behandelt. Einengen im Vakuum, Aufnehmen des Rückstandes in Chloroform, Waschen mit Salzsäure und Natronlauge, Trocknen, Einengen und Umkristallisation des Rückstandes aus Ethanol ergibt 8,2 g 6-Nitro-5-phenoxy-1-indanon vom Schmelzpunkt 103 °C.

c) 10,2 g dieser Verbindung werden reduziert wie in Beispiel 29c beschrieben. 5,7 g 6-Amino-5-phenoxy-1-indanon vom Schmelzpunkt 133 °C werden erhalten.

d) 4,57 g dieser Verbindung werden mit Methansulfonylchlorid umgesetzt wie in Beispiel 29d beschrieben. 5,9 g 6-Methylsulfonylamino-5-phenoxy-1-indanon vom Schmelzpunkt 156 °C werden erhalten.

Beispiel 45

Analog Beispiel 38 wird aus 6-Methylsulfonylamino-5-phenoxy-1-indanon N-(1-Hydroximino-5-phenoxy-6-indanyl)-methansulfonamid vom Schmelzpunkt 200 °C erhalten.

Beispiel 46

Analog Beispiel 37 wird aus 6-Methylsulfonylamino-5-phenoxy-1-indanon erhalten: 6-Methylsulfonylamino-5-phenoxy-1-indanol vom Schmelzpunkt 156 °C.

Beispiel 47

Analog Beispiel 44 wird aus 6-Methylsulfonylamino-5-phenoxy-1-indanol erhalten: N-(6-Phenoxy-5-indenyl)-methansulfonamid vom Schmelzpunkt 126 °C.

Beispiel 48

a) 1,56 g 5-Fluor-6-nitro-1-indanon werden in 28 ml Dimethylsulfoxid 30 Minuten bei 20 °C mit 0,8 g Natriumhydrogencarbonat und 0,88 g Thiophenol behandelt. Einengen im Vakuum, Aufnehmen des Rückstandes in Chloroform, Waschen mit Salzsäure und Natronlauge, Trocknen, Einengen und Umkristallisation des Rückstandes aus Ethanol ergibt 1,27 g 6-Nitro-5-phenylthio-1-indanon vom Schmelzpunkt 146 °C.

b) 0,52 g dieser Verbindung werden reduziert wie in Beispiel 29c beschrieben. 153 mg 6-Amino-5-phenylthio-1-indanon vom Schmelzpunkt 142 °C werden erhalten.

c) 250 mg dieser Verbindung werden mit Methansulfonylchlorid umgesetzt wie in Beispiel 29d beschrieben. 270 mg 6-Methylsulfonylamino-5-phenylthio-1-indanon vom Schmelzpunkt 166 °C werden erhalten.

Beispiel 49

0,91 g N-(6-Phenoxy-5-indanyl)-methansulfonamid werden in 3 ml Essigsäure und 0,72 ml Essigsäureanhydrid gelöst und bei 5 bis 10 °C mit einer Lösung von 0,39 ml Chrom-VI-oxid in 0,3 ml Wasser und 2 ml Essigsäure versetzt. Nach 60 Stunden bei 20 °C wird auf Wasser gegossen und die Substanz mit Essigester extrahiert. Neutralwaschen und Einengen der Lösung ergibt ein Substanzgemisch. Durch Chromatografie über Kieselgel werden 0,47 g 5-Methylsulfonylamino-6-phenoxy-1-indanon vom Schmelzpunkt 175 °C erhalten.

Beispiel 50

740 mg 5-Methylsulfonylamino-6-phenoxy-1-indanol, 7 ml Pyridin und 203 mg Acetylchlorid werden 2 Stunden bei 20 °C und 1 Stunde bei 60 °C gerührt. Nach Abdestillieren des Pyridins wird mit Eiswasser versetzt, mit Salzsäure angesäuert und mit Chloroform extrahiert. Einengen der Chloroformlösung und Chromatografie des Rückstandes über 30 g Kieselgel mit Chloroform ergibt zunächst 280 mg N-Acetyl-N-(1-acetoxy-6-phenoxy-5-indanyl)-methansulfonamid vom Schmelzpunkt 148 °C und danach 200 mg N-(1-Acetoxy-6-phenoxy-5-indanyl)-methansulfonamid vom Schmelzpunkt 70 °C.

## Patentansprüche

1. Indanyl-Derivate der allgemeinen Formel I

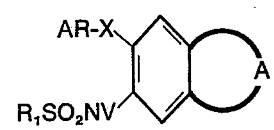

(I),

worin

AR einen gegebenenfalls durch 1 bis 3 Halogenatome, 1 bis 3, 1 bis 4 Kohlenstoffatome enthaltende Alkylgruppen oder 1 bis 3 Trifluormethylgruppen substituierter Phenylrest oder Pyridylrest,

X ein Sauerstoffatom oder ein Schwefelatom,

$R_1$ eine gegebenenfalls durch Fluoratome oder Chloratome substituierte, 1 bis 4 Kohlenstoffatome enthaltende Alkylgruppe,

V ein Wasserstoffatom, eine Acylgruppe mit 1 bis 6 Kohlenstoffatomen oder die Gruppierung $R_1SO_2-$ mit $R_1$ in der oben genannten Bedeutung, und

A die Gruppierungen

$-CH_2-CH_2-CH_2-$, $-CH_2=CH-CH_2-$, $\overset{\overset{Y}{\parallel}}{-C}-CH_2-CH_2-$, $\overset{\overset{Z}{|}}{-CH}-CH_2-CH_2-$,

$\overset{\overset{Y}{\parallel}}{-C}-\overset{\overset{SOnR_2}{|}}{CH}-CH_2-$ oder $\overset{\overset{Z}{|}}{-CH}-\overset{\overset{SOnR_2}{|}}{CH}-CH_2-$, worin

Y eine Oxogruppe, eine Oximinogruppe, eine Alkoximinogruppe mit 1 bis 4 Kohlenstoffatomen, eine Phenylhydrazonogruppe oder eine p-Toluolsulfonylhydrazonogruppe,

Z eine Hydroxygruppe, eine Acyloxygruppe mit 1 bis 6 Kohlenstoffatomen, eine Aminogruppe, eine Acylaminogruppe mit 1 bis 6 Kohlenstoffatomen oder die Gruppierungen $R_1SO_3-$ oder $R_1SO_2NH-$ mit $R_1$ in der oben genannten Bedeutung,

n die Ziffern 0, 1 oder 2 und

$R_2$ eine 1 bis 4 Kohlenstoffatome enthaltende Alkylgruppe oder eine gegebenenfalls durch Halogenatome, 1 bis 4 Kohlenstoffatome enthaltende Alkylgruppen, Nitrogruppen oder Carboxylgruppen substituierte Phenylgruppe darstellen,

bedeuten, und deren Salze mit physiologisch unbedenklichen Basen oder Säuren.

2. Indanyl-Derivate der allgemeinen Formel I gemäss Anspruch 1, dadurch gekennzeichnet, dass AR einen gegebenenfalls durch Halogenatome, 1 bis 4 Kohlenstoffatome enthaltende Alkylgruppen oder Trifluormethylgruppen substituierten Phenylrest und A die Gruppierung $-CH_2CH_2CH_2-$ darstellen.

3. Indanyl-Derivate der allgemeinen Formel I gemäss Anspruch 1, dadurch gekennzeichnet, dass AR einen gegebenenfalls durch ein oder zwei Fluoratome oder Chloratome substituierter Phenylrest darstellt.

4. Indanyl-Derivate der allgemeinen Formel I gemäss Ansprüchen 1 und 3, dadurch gekennzeichnet, dass der Substituent $R_1$ eine Methylgruppe darstellt.

5. Indanyl-Derivate der allgemeinen Formel I gemäss Ansprüchen 1, 3 und 4, dadurch gekennzeichnet, dass Y eine Oxogruppe darstellt.

6. Pharmazeutische Präparate, gekennzeichnet durch den Gehalt an einem Indanyl-Derivat der allgemeinen Formel I gemäss Anspruch 1.

7. Verfahren zur Herstellung von Indanyl-Derivaten der allgemeinen Formel I gemäss Anspruch 1, dadurch gekennzeichnet, dass man

a) eine Verbindung der allgemeinen Formel II

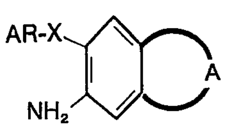

(II),

worin AR, X und A die im Anspruch 1 genannte Bedeutung besitzen, mit einem Sulfonsäurederivat der allgemeinen Formel III

$$R_1SO_2W \quad \text{(III)},$$

worin $R_1$ die in Anspruch 1 genannte Bedeutung besitzt und W ein Halogenatom oder den Rest $R_1SO_2O-$ darstellt, kondensiert, oder

b) zur Herstellung von Indan-Derivaten der allgemeinen Formel I mit A in der Bedeutung von $-COCH_2CH_2-$ ein Indan-Derivat der allgemeinen Formel I mit A in der Bedeutung von $-CH_2CH_2CH_2-$ oxydiert, oder

c) zur Herstellung von Indan-Derivaten der allgemeinen Formel I mit A in der Bedeutung von

$$-CO-\underset{}{\overset{SOnR_2}{\overset{|}{CH}}}-CH_2-$$

ein Indan-Derivat der allgemeinen Formel I mit A in der Bedeutung von $-COCH_2CH_2-$ in Gegenwart starker Basen mit einem Disulfid der allgemeinen Formel IV

$$R_2-S-S-R_2 \quad \text{(IV)},$$

worin $R_2$ die oben genannte Bedeutung besitzt, umsetzt und gegebenenfalls die erhaltenen Thioverbindungen der allgemeinen Formel I zu den entsprechenden Sulfoxiden oder Sulfonen oxydiert, oder

d) zur Herstellung von Indan-Derivaten der allgemeinen Formel I mit Y in der Bedeutung einer Oximinogruppe, einer Alkoximinogruppe mit 1 bis 4 Kohlenstoffatomen, einer Phenylhydrazonogruppe oder einer p-Toluolsulfonylhydrazonogruppe ein Indan-Derivat der allgemeinen Formel I mit Y in der Bedeutung einer Oxogruppe mit dem entsprechenden Oxim oder Hydrazin kondensiert, oder

e) zur Herstellung eines Indan-Derivats der allgemeinen Formel I mit Z in der Bedeutung einer Hydroxygruppe oder einer Aminogruppe ein Indan-Derivat der allgemeinen Formel I mit A in der Bedeutung von $-\overset{Y}{\overset{\|}{C}}-CH_2CH_2-$ oder $-\overset{Y}{\overset{\|}{C}}-\overset{SOnR_2}{\overset{|}{CH}}-CH_2-$ reduziert, oder

f) zur Herstellung von Indan-Derivaten der allgemeinen Formel I mit A in der Bedeutung von $-CH=CH-CH_2-$ ein Indan-Derivat der allgemeinen Formel I mit A in der Bedeutung von $-\overset{OH}{\overset{|}{CH}}-CH_2CH_2-$ dehydratisiert, und gegebenenfalls die Indan-Derivate mit V in der Bedeutung von Wasserstoff und/oder Z in der Bedeutung einer Hydroxygruppe oder einer Aminogruppe acyliert.

8. N-(6-Phenoxy-5-indanyl)-methansulfonamid, eine Verbindung gemäss Anspruch 1.

9. N-[6-(4-Chlorphenoxy)-5-indanyl]-methansulfonamid, eine Verbindung gemäss Anspruch 1.

10. N-[6-(4-Tolyloxy)-5-indanyl]-methansulfonamid, eine Verbindung gemäss Anspruch 1.

11. N-[6-(4-Fluorphenoxy)-5-indanyl]-methansulfonamid, eine Verbindung gemäss Anspruch 1.

12. N-[6-(4-Chlor-2-methyl-phenoxy)-5-indanyl]-methansulfonamid, eine Verbindung gemäss Anspruch 1.

13. N-(6-Phenylthio-5-indanyl)-methansulfonamid, eine Verbindung gemäss Anspruch 1.

14. N-[6-(3-Chlorphenoxy)-5-indanyl]-methansulfonamid, eine Verbindung gemäss Anspruch 1.

15. N-[6-(2-Chlorphenoxy)-5-indanyl]-methansulfonamid, eine Verbindung gemäss Anspruch 1.

16. N-[6-(2-Fluorphenoxy)-5-indanyl]-methansulfonamid, eine Verbindung gemäss Anspruch 1.

17. N-[6-(2-Chlor-4-fluorphenoxy)-5-indanyl]-methansulfonamid, eine Verbindung gemäss Anspruch 1.

18. N-[6-(3,4-Dichlorphenoxy)-5-indanyl]-methansulfonamid, eine Verbindung gemäss Anspruch 1.

19. N-[6-(4-Bromphenoxy)-5-indanyl]-methansulfonamid, eine Verbindung gemäss Anspruch 1.

20. N-[6-(2,4-Dichlorphenoxy)-5-indanyl]-methansulfonamid, eine Verbindung gemäss Anspruch 1.

21. N-[6-(2-Tolyloxy)-5-indanyl]-methansulfonamid, eine Verbindung gemäss Anspruch 1.

22. N-[6-(3-Fluorphenoxy)-5-indanyl]-methansulfonamid, eine Verbindung gemäss Anspruch 1.

23. N-(6-Phenylthio-5-indanyl)-methansulfonamid, eine Verbindung gemäss Anspruch 1.

24. N-[6-(4-tert.-Butylphenylthio)-5-indanyl]-methansulfonamid, eine Verbindung gemäss Anspruch 1.

25. N-[6-(4-Fluorphenylthio)-5-indanyl]-methansulfonamid, eine Verbindung gemäss Anspruch 1.

26. N-[6-(4-Chlorphenylthio)-5-indanyl]-methansulfonamid, eine Verbindung gemäss Anspruch 1.

27. N-[6-(2-Pyridylthio)-5-indanyl]-methansulfonamid, eine Verbindung gemäss Anspruch 1.

28. N-[6-(4-Pyridylthio)-5-indanyl]-methansulfonamid, eine Verbindung gemäss Anspruch 1.

29. N-[6-(3-Pyridyloxy)-5-indanyl]-methansulfonamid, eine Verbindung gemäss Anspruch 1.

30. N-(6-Phenoxy-5-indanyl)-trifluormethansulfonamid, eine Verbindung gemäss Anspruch 1.

31. N-(6-Phenylthio-5-indanyl)-trifluormethansulfonamid, eine Verbindung gemäss Anspruch 1.

32. N-[6-(4-Fluorphenoxy)-5-indanyl]-trifluormethansulfonamid, eine Verbindung gemäss Anspruch 1.

33. N-[6-(4-Chlorphenoxy)-5-indanyl]-trifluormethansulfonamid, eine Verbindung gemäss Anspruch 1.

34. N-(6-Phenoxy-5-indanyl)-chlormethansulfonamid, eine Verbindung gemäss Anspruch 1.

35. N-(6-Phenoxy-5-indanyl)-äthansulfonamid, eine Verbindung gemäss Anspruch 1.

36. 6-(4-Chlorphenoxy)-5-methylsulfonylamino-1-indanon, eine Verbindung gemäss Anspruch 1.

37. 6-(4-Fluorphenoxy)-5-methylsulfonylamino-1-indanon, eine Verbindung gemäss Anspruch 1.

38. 6-(3-Chlorphenoxy)-5-methylsulfonylamino-1-indanon, eine Verbindung gemäss Anspruch 1.

39. 6-(2-Fluorphenoxy)-5-methylsulfonylamino-1-indanon, eine Verbindung gemäss Anspruch 1.

40. 5-Methylsulfonylamino-6-phenoxy-2-phenylthio-1-indanon, eine Verbindung gemäss Anspruch 1.

41. 5-Methylsulfonylamino-6-phenoxy-2-phenylsulfinyl-1-indanon, eine Verbindung gemäss Anspruch 1.

42. 5-Methylsulfonylamino-6-phenoxy-2-phenylsulfonyl-1-indanon, eine Verbindung gemäss Anspruch 1.

43. 5-Methylsulfonylamino-6-phenoxy-indanol, eine Verbindung gemäss Anspruch 1.

44. N-(1-Hydroximino-6-phenoxy-5-indanyl)-methansulfonamid, eine Verbindung gemäss Anspruch 1.

45. N-(1-Methoximino-6-phenoxy-5-indanyl)-methansulfonamid, eine Verbindung gemäss Anspruch 1.

46. N-[1-(4-Toluolsulfonylhydrazono)-6-phenoxy-5-indanyl]-methansulfonamid, eine Verbindung gemäss Anspruch 1.

47. 5-Methylsulfonylamino-6-phenoxy-1-indanyllamin, eine Verbindung gemäss Anspruch 1.

48. 5-Methylsulfonylamino-6-phenoxy-2-phenylthio-1-indanol, eine Verbindung gemäss Anspruch 1.

49. N-(5-Phenoxy-6-indenyl)-methansulfonamid, eine Verbindung gemäss Anspruch 1.

50. 6-Methylsulfonylamino-5-phenoxy-1-indanon, eine Verbindung gemäss Anspruch 1.

51. N-(1-Hydroximino-5-phenoxy-6-indanyl)-methansulfonamid, eine Verbindung gemäss Anspruch 1.

52. 6-Methylsulfonylamino-5-phenoxy-1-indanol, eine Verbindung gemäss Anspruch 1.

53. N-(6-Phenoxy-5-indenyl)-methansulfonamid, eine Verbindung gemäss Anspruch 1.

54. 6-Methylsulfonylamino-5-phenylthio-1-indanon, eine Verbindung gemäss Anspruch 1.

55. 5-Methylsulfonylamino-6-phenoxy-1-indanon, eine Verbindung gemäss Anspruch 1.

56. N-Acetyl-N-(1-acetoxy-6-phenoxy-5-indanyl)methansulfonamid, eine Verbindung gemäss Anspruch 1.

57. N-(1-Acetoxy-6-phenoxy-5-indanyl)-methansulfonamid, eine Verbindung gemäss Anspruch 1.

**Claims**

1. Indanyl derivatives of the general formula I

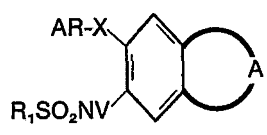

(I),

in which

AR represents a phenyl radical or pyridyl radical each optionally substituted by from 1 to 3 halogen atoms, from 1 to 3 alkyl groups containing from 1 to 4 carbon atoms or by from 1 to 3 trifluoromethyl groups,

X represents an oxygen atom or a sulphur atom,

$R_1$ represents an alkyl group containing from 1 to 4 carbon atoms which is optionally substituted by fluorine atoms or chlorine atoms,

V represents a hydrogen atom, an acyl group having from 1 to 6 carbon atoms or the grouping $R_1SO_2$ in which $R_1$ has the meaning given above, and

A represents the groupings

$-CH_2-CH_2-CH_2-$, $-CH_2=CH-CH_2-$, $-\overset{\overset{Y}{\|}}{C}-CH_2-CH_2-$, $-\overset{\overset{Z}{|}}{C}H-CH_2-CH_2-$,

$-\overset{\overset{Y}{\|}}{C}-\overset{\overset{SOnR_2}{|}}{C}H-CH_2-$ oder $-\overset{\overset{Z}{|}}{C}H-\overset{\overset{SOnR_2}{|}}{C}H-CH_2-$, in which

Y represents an oxo group, an oxyimino group, an alkoxyimino group having from 1 to 4 carbon atoms, a phenylhydrazono group or a p-toluenesulphonylhydrazono group,

Z represents a hydroxy group, an acyloxy group having from 1 to 6 carbon atoms, an amino group, an acylamino group having from 1 to 6 carbon atoms or the groupings $R_1SO_3$– or $R_1SO_2NH$– in which $R_1$ has the meaning given above,

n represents the numbers 0, 1 or 2, and

$R_2$ represents an alkyl group containing from 1 to 4 carbon atoms or represents a phenyl group optionally substituted by halogen atoms, alkyl groups containing from 1 to 4 carbon atoms, nitro groups or carboxy groups, and the salts thereof with physiologically tolerable bases or acids.

2. Indanyl derivatives of the general formula I according to claim 1, characterised in that AR represents a phenyl radical optionally substituted by halogen atoms, alkyl groups containing from 1 to 4 carbon atoms or trifluoromethyl groups, and A represents the grouping $-CH_2-CH_2-CH_2-$.

3. Indanyl derivatives of the general formula I according to claim 1, characterised in that AR represents a phenyl radical optionally substituted by one or two fluorine atoms or chlorine atoms.

4. Indanyl derivatives of the general formula I according to claims 1 and 3, characterised in that the substituent $R_1$ represents a methyl group.

5. Indanyl derivatives of the general formula I according to claims 1, 3 and 4, characterised in that Y represents an oxo group.

6. Pharmaceutical preparations characterised by a content of an indanyl derivative of the general formula I according to claim 1.

7. Process for the manufacture of indanyl derivatives of the general formula I according to claim 1, characterised in that

a) a compound of the general formula II

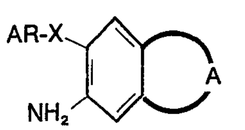

(II),

in which AR, X and A have the meanings given in claim 1 are condensed with a sulphonic acid derivative of the general formula III

$$R_1SO_2W \qquad (III)$$

in which $R_1$ has the meaning given in claim 1 and W represents a halogen atom or the radical $R_1SO_2O$–, or

b) for the manufacture of indane derivatives of the general formula I in which A represents $-COCH_2CH_2-$, an indane derivative of the general formula I in which A represents $-CH_2CH_2CH_2-$ is oxidised, or

c) for the manufacture of indane derivatives of the general formula I in which A represents

SOnR$_2$
|
$-CO-CH-CH_2-$, an indane derivative of the general formula I in which A represents $-COCH_2CH_2-$ is reacted in the presence of strong bases with a disulphide of the general formula IV

$$R_2-S-S-R_2 \qquad (IV)$$

in which $R_2$ has the meaning given above and, optionally, the resulting thio compounds of the general formula I are oxidised to form the corresponding sulphoxides or sulphones, or

d) for the manufacture of indane derivatives of the general formula I in which Y represents an oxyimino group, an alkoxyimino group having from 1 to 4 carbon atoms, a phenylhydrazono group or a p-toluenesulphonylhydrazono group, an indane derivative of the general formula I in which Y represents an oxo group is condensed with the corresponding oxime or hydrazine, or

e) for the manufacture of an indane derivative of the general formula I in which Z represents a hydroxy group or an amino group, an indane derivative of the general formula I in which A represents

Y Y SOn-R$_2$
presents $-\overset{\|}{C}-CH_2-CH_2-$ or $-\overset{\|}{C}-\overset{|}{C}H-CH_2-$ is reduced, or

f) for the manufacture of indane derivatives of the general formula I in which A represents $-CH=CH-CH_2-$, an indane derivative of the general formula I in which A represents

OH
|
$-CH-CH_2CH_2-$ is dehydrated and, optionally, the indane derivatives in which V represents hydrogen and/or Z represents a hydroxy group or an amino group are acylated.

8. N-(6-phenoxy-5-indanyl)-methanesulphonamide, a compound according to claim 1.

9. N-[6-(4-chlorophenoxy)-5-indanyl]-methanesulphonamide, a compound according to claim 1.

10. N-[6-(4-tolyloxy)-5-indanyl]-methanesulphonamide, a compound according to claim 1.

11. N-[6-(4-fluorophenoxy)-5-indanyl]-methanesulphonamide, a compound according to claim 1.

12. N-[6-(4-chloro-2-methylphenoxy)-5-indanyl]-methanesulphonamide, a compound according to claim 1.

13. N-(6-phenylthio-5-indanyl)-methanesulphonamide, a compound according to claim 1.

14. N-[6-(3-chlorophenoxy)-5-indanyl]-methanesulphonamide, a compound according to claim 1.

15. N-[6-(2-chlorophenoxy)-5-indanyl]-methanesulphonamide, a compound according to claim 1.

16. N-[6-(2-fluorophenoxy)-5-indanyl]-methanesulphonamide, a compound according to claim 1.

17. N-[6-(2-chloro-4-fluorophenoxy)-5-indanyl]-methanesulphonamide, a compound according to claim 1.

18. N-[6-(3,4-dichlorophenoxy)-5-indanyl]-methanesulphonamide, a compound according to claim 1.

19. N-[6-(4-bromophenoxy)-5-indanyl]-methanesulphonamide, a compound according to claim 1.

20. N-[6-(2,4-dichlorophenoxy)-5-indanyl]-methanesulphonamide, a compound according to claim 1.

21. N-[6-(2-tolyloxy)-5-indanyl]-methanesulphonamide, a compound according to claim 1.

22. N-[6-(3-fluorophenoxy)-5-indanyl]-methanesulphonamide, a compound according to claim 1.

23. N-(6-phenylthio-5-indanyl)-methanesulphonamide, a compound according to claim 1.

24. N-[6-(4-tert.-butylphenylthio)-5-indanyl]-methanesulphonamide, a compound according to claim 1.

25. N-[6-(4-fluorophenylthio)-5-indanyl]-methanesulphonamide, a compound according to claim 1.

26. N-[6-(4-chlorophenylthio)-5-indanyl]-methanesulphonamide, a compound according to claim 1.

27. N-[6-(2-pyridylthio)-5-indanyl)-methanesulphonamide, a compound according to claim 1.

28. N-[6-(4-pyridylthio)-5-indanyl]-methanesulphonamide, a compound according to claim 1.

29. N-[6-(3-pyridyloxy)-5-indanyl]-methanesulphonamide, a compound according to claim 1.

30. N-(6-phenoxy-5-indanyl)-trifluoromethanesulphonamide, a compound according to claim 1.

31. N-(6-phenylthio-5-indanyl)-trifluoromethanesulphonamide, a compound according to claim 1.

32. N-[6-(4-fluorophenoxy)-5-indanyl]-trifluoromethanesulphonamide, a compound according to claim 1.

33. N[6-(4-chlorophenoxy)-5-indanyl]-trifluoromethanesulphonamide, a compound according to claim 1.

34. N-(6-phenoxy-5-indanyl)-chloromethanesulphonamide, a compound according to claim 1.

35. N-(6-phenoxy-5-indanyl)-ethanesulphonamide, a compound according to claim 1.

36. 6-(4-chlorophenoxy)-5-methylsulphonylamino-1-indanone, a compound according to claim 1.

37. 6-(4-fluorophenoxy)-5-methylsulphonylamino-1-indanone, a compound according to claim 1.

38. 6-(3-chlorophenoxy)-5-methylsulphonylamino-1-indanone, a compound according to claim 1.

39. 6-(2-fluorophenoxy)-5-methylsulphonylamino-1-indanone, a compound according to claim 1.

40. 5-methylsulphonylamino-6-phenoxy-2-phenylthio-1-indanone, a compound according to claim 1.

41. 5-methylsulphonylamino-6-phenoxy-2-phenylsulphinyl-1-indanone, a compound according to claim 1.

42. 5-methylsulphonylamino-6-phenoxy-2-phenylsulphonyl-1-indanone, a compound according to claim 1.

43. 5-methylsulphonylamino-6-phenoxyindanol, a compound according to claim 1.

44. N-(1-hydroxyimino-6-phenoxy-5-indanyl)-methanesulphonamide, a compound according to claim 1.

45. N-(1-methoxyimino-6-phenoxy-5-indanyl)-methanesulphonamide, a compound according to claim 1.

46. N-[1-(4-toluenesulphonylhydrazono)-6-phenoxy-5-indanyl]-methanesulphonamide, a compound according to claim 1.

47. 5-methylsulphonylamino-6-phenoxy-1-indanylamine, a compound according to claim 1.

48. 5-methylsulphonylamino-6-phenoxy-2-phenylthio-1-indanol, a compound according to claim 1.

49. N-(5-phenoxy-6-indenyl)-methanesulphonamide, a compound according to claim 1.

50. 6-methylsulphonylamino-5-phenoxy-1-indanone, a compound according to claim 1.

51. N-(1-hydroxyimino-5-phenoxy-6-indanyl)-methanesulphonamide, a compound according to claim 1.

52. 6-methylsulphonylamino-5-phenoxy-1-indanol, a compound according to claim 1.

53. N-(6-phenoxy-5-indenyl)-methanesulphonamide, a compound according to claim 1.

54. 6-methylsulphonylamino-5-phenylthio-1-indanone, a compound according to claim 1.

55. 5-methylsulphonylamino-6-phenoxy-1-indanone, a compound according to claim 1.

56. N-acetyl-N-(1-acetoxy-6-phenoxy-5-indanyl)-methanesulphonamide, a compound according to claim 1.

57. N-(1-acetoxy-6-phenoxy-5-indanyl)-methanesulphonamide, a compound according to claim 1.

## Revendications

1. Dérivés de l'indane répondant à la formule générale I

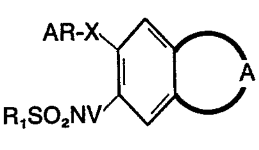

(I),

dans laquelle

AR représente un radical phényle ou pyridyle portant éventuellement de 1 à 3 atomes d'halogène, de 1 à 3 radicaux alkyles renfermant de 1 à 4 atomes de carbone ou de 1 à 3 radicaux trifluorométhyles,

X représente un atome d'oxygène ou un atome de soufre,

$R_1$ représente un radical alkyle contenant de 1 à 4 atomes de carbone et éventuellement porteur d'atomes de fluor ou d'atomes de chlore,

V représente un atome d'hydrogène, un radical acyle contenant de 1 à 6 atomes de carbone ou un groupement $R_1SO_2-$ dans lequel $R_1$ a la signification indiquée ci-dessus, et

A représente un groupement répondant à l'une des formules:

$-CH_2-CH_2-CH_2-$, $-CH_2=CH-CH_2-$, $-\overset{Y}{\overset{\|}{C}}-CH_2-CH_2-$, $-\overset{Z}{\overset{|}{C}}H-CH_2-CH_2-$,

$-\overset{Y}{\overset{\|}{C}}-\overset{SOnR_2}{\overset{|}{C}}H-CH_2-$ oder $-\overset{Z}{\overset{|}{C}}H-\overset{SOnR_2}{\overset{|}{C}}H-CH_2-$, dans lesquelles:

Y représente un radical oxo, un radical oximino, un radical alcoximino contenant de 1 à 4 atomes de carbone, un radical phénylhydrazono ou un radical p-toluène-sulfonylhydrazono,

Z représente un groupe hydroxy, un radical acyloxy contenant de 1 à 6 atomes de carbone, un radical amino, un radical acylamino contenant de 1 à 6 atomes de carbone ou un groupement répondant à l'une des formules $R_1SO_3-$ et $R_1SO_2NH-$ dans lesquelles $R_1$ a la signification indiquée cidessus,

n représente l'un des nombres 0, 1 et 2 et

$R_2$ représente un radical alkyle contenant de 1 à 4 atomes de carbone ou un radical phényle éventuellement porteur d'atomes d'halogènes, de radicaux alkyles contenant de 1 à 4 atomes de carbone, de groupes nitro ou de groupes carboxy, et les sels qu'ils forment avec des bases ou des acides inoffensifs du point de vue physiologique.

2. Dérivés de l'indane de formule générale I selon la revendication 1, caractérisés en ce que: AR représente un radical phényle éventuellement porteur d'atomes d'halogènes, de radicaux alkyles contenant de 1 à 4 atomes de carbone ou de radicaux trifluorométhyles, et A représente le groupement $-CH_2CH_2CH_2-$.

3. Dérivés de l'indane de formule générale I selon la revendication 1, caractérisés en ce que: AR représente un radical phényle éventuellement porteur d'un ou deux atomes de fluor ou de chlore.

4. Dérivés de l'indane de formule générale I selon l'une des revendications 1 et 3, caractérisés en ce que le substituant $R_1$ est un radical méthyle.

5. Dérivés de l'indane de formule générale I selon l'une quelconque des revendications 1, 3 et 4, caractérisés en ce que: Y représente un groupe oxo.

6. Compositions pharmaceutiques caractérisées en ce qu'elles contiennent un dérivé de l'indane de formule générale I selon la revendication 1.

7. Procédé de préparation de dérivés de l'indane de formule générale I selon la revendication 1, procédé caractérisé en ce que:

a) On condense un composé répondant à la formule générale II

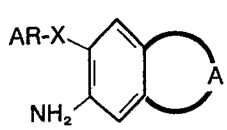

(II),

dans laquelle AR, X et A ont les significations données à la revendication 1, avec un dérivé d'acide sulfonique répondant à la formule générale III

$$R_1SO_2W \qquad (III)$$

dans laquelle $R_1$ a la signification donnée à la revendication 1 et W représente un atome d'halogène ou un radical $R_1SO_2O-$, ou,

b) pour préparer des dérivés de l'indane de formule générale I dans lesquels A représente un radical $-COCH_2CH_2-$ on oxyde un dérivé de l'indane de formule générale I dans lequel A représente un radical $-CH_2CH_2CH_2-$, ou,

c) pour préparer des dérivés de l'indane de formule générale I dans lesquels A représente un radical

SOnR₂
|
–CO–CH–CH₂

on fait réagir un dérivé de l'indane de formule générale I dans lequel A représente un radical $-COCH_2CH_2-$, en présence de bases fortes, avec un disulfure répondant à la formule générale IV

$$R_2-S-S-R_2 \qquad (IV)$$

dans laquelle $R_2$ a la signification donnée cidessus, et on oxyde éventuellement les composés thio obtenus, qui répondent à la formule générale I, en les sulfoxydes correspondants ou les sulfones correspondantes, ou

d) pour préparer des dérivés de l'indane de formule générale I dans lesquels Y représente un radical hydroximino, un radical alcoximino contenant de 1 à 4 atomes de carbone, un radical phénylhydrazono ou un radical p-toluène-sulfonylhydrazono, on condense un dérivé de l'indane de formule générale I dans lequel Y représente un groupe oxo avec l'oxime correspondante ou l'hydrazine correspondante, ou,

e) pour préparer un dérivé de l'indane de formule générale I dans lequel Z représente un groupe hydroxy ou un groupe amino, on réduit un dérivé de l'indane de formule générale I dans lequel A représente un radical

Y
‖
–C–CH₂CH₂–

Y SOnR₂
‖ |
–C–CH–CH₂–

ou,

f) pour préparer des dérivés de l'indane de formule générale I dans lesquels A représente un radical $-CH=CH-CH_2-$, on déshydrate un dérivé de l'indane de formule générale I dans lequel A représente un radical

OH
|
–CH–CH₂CH₂–

et éventuellement on acyle les dérivés de l'indane dans lesquels V représente l'hydrogène et/ou Z représente un groupe hydroxy ou un groupe amino.

8. Composé selon la revendication 1, en l'espèce: le N-(phénoxy-6 indanyl-5)-méthane-sulfonamide.

9. Composé selon la revendication 1, en l'espèce: le N-[(chloro-4-phénoxy)-6 indanyl-5]-méthane-sulfonamide.

10. Compsé selon la revendication 1, en l'espèce: le N-[(tolyl-4 oxy)-6 indanyle-5]-méthane-sulfonamide.

11. Composé selon la revendication 1, en l'espèce: le N-[(fluoro-4 phénoxy)-6 indanyl-5]-méthane-sulfonamide.

12. Composé selon la revendication 1, en l'espèce: le N-[(chloro-4 méthyl-2-phénoxy)-6 indanyl-5]-méthane-sulfonamide.

13. Composé selon la revendication 1, en l'espèce: le N-(phénylthio-6 indanyl-5)-méthane-sulfonamide.

14. Composé selon la revendication 1, en l'espèce: le N-[(chloro-3 phénoxy)-6 indanyl-5]-méthane-sulfonamide.

15. Composé selon la revendication 1, en l'espèce: le N-[(chloro-2 phénoxy)-6 indanyl-5]-méthane-sulfonamide.

16. Composé selon la revendication 1, en l'espèce: le N-[(fluoro-2-phénoxy)-6 indanyl-5]-méthane-sulfonamide.

17. Composé selon la revendication 1, en l'espèce: le N-[(chloro-2-fluoro-4 phénoxy)-6 indanyl-5]-méthane-sulfonamide.

18. Composé selon la revendication 1, en l'espèce: le N-[(dichloro-3,4 phénoxy)-6 indanyl-5]-méthane-sulfonamide.

19. Composé selon la revendication 1, en l'espèce: le N-[(bromo-4 phénoxy)-6 indanyl-5]-méthane-sulfonamide.

20. Composé selon la revendication 1, en l'espèce: le N-[(dichloro-2,4 phénoxy)-6 indanyl-5]-méthane-sulfonamide.

21. Composé selon la revendication 1, en l'espèce: le N-[(tolyl-2 oxy)-6 indanyl-5]-méthane-sulfonamide.

22. Composé selon la revendication 1, en l'espèce: le N-[(fluoro-3-phénoxy)-6 indanyl-5]-méthane-sulfonamide.

23. Composé selon la revendication 1, en l'espèce: le N-(phénylthio-6 indanyl-5)-méthane-sulfonamide.

24. Composé selon la revendication 1, en l'espèce: le N-[(tert-butyl-4 phénylthio)-6 indanyl-5]-méthane-sulfonamide.

25. Composé selon la revendication 1, en l'espèce: le N-[(fluoro-4 phénylthio)-6 indanyl-5]-méthane-sulfonamide.

26. Composé selon la revendication 1, en l'espèce: le N-[(chloro-4 phénylthio)-6 indanyl-5]-méthane-sulfonamide.

27. Composé selon la revendication 1, en l'espèce: le N-[(pyridyl-2 thio)-6 indanyl-5]-méthane-sulfonamide.

28. Composé selon la revendication 1, en l'espèce: le N-[(pyridyl-4 thio)-6 indanyl-5]-méthane-sulfonamide.

29. Composé selon la revendication 1, en l'espèce: le N-[(pyridyl-3 oxy)-6 indanyl-5]-méthane-sulfonamide.

30. Composé selon la revendication 1, en l'espèce: le N-(phénoxy-6 indanyl-5)-trifluorométhane-sulfonamide.

31. Composé selon la revendication 1, en l'espèce: le N-(phénylthio-6 indanyl-5)-trifluorométhane-sulfonamide.

32. Compsé selon la revendication 1, en l'espèce: le N-[(fluoro-4 phénoxy)-6 indanyl-5]-trifluoro-méthane-sulfonamide.

33. Composé selon la revendication 1, en l'espèce: le N-[(chloro-4 phénoxy)-6 indanyl-5]-trifluoro-méthane-sulfonamide.

34. Composé selon la revendication 1, en l'espèce: le N-(phénoxy-6 indanyl-5)-chlorométhane-sulfonamide.

35. Composé selon la revendication 1, en l'espèce: le N-(phénoxy-6 indanyl-5)-éthane-sulfonamide.

36. Composé selon la revendication 1, en l'espèce: la (chloro-4 phénoxy)-6 méthylsulfonylamino-5 indanone-1.

37. Composé selon la revendication 1, en l'espèce: la (fluoro-4 phénoxy)-6 méthylsulfonylamino-5 indanone-1.

38. Composé selon la revendication 1, en l'espèce: la (chloro-3 phénoxy)-6 méthylsulfonylamino-5 indanone-1.

39. Composé selon la revendication 1, en l'espèce: la (fluoro-2 phénoxy)-6 méthylsulfonylamino-5 indanone-1.

40. Composé selon la revendication 1, en l'espèce: la méthylsulfonylamino-5 phénoxy-6 phénylthio-2 indanone-1.

41. Composé selon la revendication 1, en l'espèce: la méthylsulfonylamino-5 phénoxy-6 phénylsulfinyl-2 indanone-1.

42. Composé selon la revendication 1, en l'espèce: la méthylsulfonylamino-5 phénoxy-6 phénylsulfonyl-2 indanone-1.

43. Composé selon la revendication 1, en l'espèce: la méthylsulfonylamino-5 phénoxy-6 indanol-1.

44. Composé selon la revendication 1, en l'espèce: le N-(hydroximino-1 phénoxy-6 indanyl-5)-méthane-sulfonamide.

45. Composé selon la revendication 1, en l'espèce: le N-(méthoximino-1 phénoxy-6 indanyl-5)-méthane-sulfonamide.

46. Composé selon la revendication 1, en l'espèce: le N-[(p-toluène-sulfonyl-hydrazono)-1 phénoxy-6 indanyl-5]-méthane-sulfonamide.

47. Composé selon la revendication 1, en l'espèce: la (méthylsulfonylamino-5 phénoxy-6 indanyl-1)-amine.

48. Composé selon la revendication 1, en l'espèce: le méthylsulfonylamino-5 phénoxy-6 phénylthio-2 indanol-1.

49. Composé selon la revendication 1, en l'espèce: le N-(phénoxy-5 indényl-6)-méthane-sulfonamide.

50. Composé selon la revendication 1, en l'espèce: la méthylsulfonylamino-6 phénoxy-5 indanone-1.

51. Composé selon la revendication 1, en l'espèce: le N-(hydroximino-1 phénoxy-5 indanyl-6)-méthane-sulfonamide.

52. Composé selon la revendication 1, en l'espèce: le méthylsulfonylamino-6 phénoxy-5 indanol-1.

53. Composé selon la revendication 1, en l'espèce: le N-(phénoxy-6 indényle-5)-méthane-sulfonamide.

54. Composé selon la revendication 1, en l'espèce: la méthylsulfonylamino-6 phénylthio-5 indanone-1.

55. Composé selon la revendication 1, en l'espèce: la méthylsulfonylamino-5 phénoxy-6 indanone-1.

56. Composé selon la revendication 1, en l'espèce: le N-acétyl-N-(acétoxy-1 phénoxy-6 indanyl-5)-méthane-sulfonamide.

57. Composé selon la revendication 1, en l'espèce: le N-(acétoxy-1 phénoxy-6 indanyl-5)-méthane-sulfonamide.